# EUROPEAN PATENT SPECIFICATION

(11) **EP 1 019 078 B1**
(45) Date of publication and mention of the grant of the patent: **23.07.2003**
(21) Application number: 98917896.7
(22) Date of filing: 14.04.1998
(51) Int. Cl.: A61K 38/17, C07K 14/47, A61P 29/02

(54) **USE OF CARTILAGE OLIGOMERIC MATRIX PROTEIN FOR THE TREATMENT OF RHEUMATOID ARTHRITIS**
VERWENDUNG VON OLIGOMEREM MATRIXPROTEIN AUS KNORPEL ZUR BEHANDLUNG VON RHEUMATOIDER ARTHRITIS
UTILISATION D'UNE PROTEINE OLIGOMERIQUE DE CARTILAGE POUR LE TRAITEMENT DE LA POLYARTHRITE RHUMATOIDE

(30) Priority: 15.04.1997 SE 9701409
(43) Date of publication of application: 19.07.2000
(73) Proprietor: AstraZeneca AB, 151 85 Södertälje (SE)
(72) Inventor: HEINEGARD, Dick Lunds Universitet Dept. of Cell, Biology P.O. Box 94 S-221 00 Lund (SE); LORENTZEN, Johnny, C., S-753 25 Uppsala (SE); KLARESKOG, Lars, S-752 18 Uppsala (SE)
(74) Representative: As Sivborg, Susanne Birgitta
(86) International application number: SE9800682
(87) International publication number: WO98046253

(56) References cited:
- WO-A-91/10424
- WO-A-94/15627
- DIALOG INFORMATION SERVICES, File 155, Medline, Dialog Accession No. 08369593, BRIGGS M.D. et al., "Pseudoachondroplasia and Multiple Epiphyseal Dysplasia Due to Mutations in the Cartilage Oligomeric Matrix Protein Gene"; & NAT. GENET., (UNITED STATES), July 1995, 10(3), p. 330-6.
- DIALOG INFORMATION SERVICES, File 155, Medline, Dialog Accession No. 07153808, Medline Accession No. 92404864, FORSLIND K. et al., Increased Serum Concentrations of Cartilage Oligomeric Matrix Protein. A Prognostic Marker in Early Rheumatoid Arthritis"; & BR. J. RHEUMATOL., (ENGLAND), Sep. 1992, 31(9), p. 593-8.

## Description

### Background

The present invention relates to use of cartilage oligomeric matrix protein or fragments or peptide analogues thereof, for the manufacture of a pharmaceutical composition for the prevention or treatment of rheumatoid arthritis in mammals.

Arthritis is a common name for several inflammatory joint diseases, e.g. rheumatoid arthritis, bacterial arthritis, reactive arthritis, and crystal-induced arthritis. Rheumatoid arthritis (RA) comprises a large group of non-bacterial conditions. Classical rheumatoid arthritis normally affects several joints (polyarthritis), but may also be restricted to a single joint (mono-arthritis). The attack of the joint cartilage is only one of several factors which degenerate the cartilage and the bone and destroy the joint function. Also the joint capsule, the ligaments, and the bone tissue are affected.

### Description of the Prior Art

At present, the symptoms of e.g. rheumatoid arthritis for some patients can be alleviated and the joint function be maintained or improved by medical treatment, e.g. with corticosteroids or methotrexat, physiotherapy, or surgical treatment. However, several patients are difficult or impossible to improve by treatment. Further, there exists no method for prevention of rheumatoid arthritis.

Thus, there is a need to prevent the symptoms of rheumatoid arthritis. There is also a need of further alleviating and reducing the symptoms of rheumatoid arthritis.

### Summary of the Invention

The object of the present invention is to prevent or alleviate the symptoms of rheumatoid arthritis. This object is achieved by use of cartilage oligomer matrix protein (COMP), or fragments or peptide analogues thereof, for the manufacture of a pharmaceutical composition for the prevention or treatment of rheumatoid arthritis in mammals.

The present invention is based on the surprising observation made by the present inventors that COMP is arthritogenic. Rheumatoid arthritis is an autoimmune disease, i.e. it is considered to be due to a malfunction of the immune system of mammals wherein the immune system fails to distinguish between foreign substances exposed to the mammal and the various endogenous substances of the mammal and begins to make antibodies to the latter.

Cartilage oligomer matrix protein (COMP) is an 524 kDa oligomeric glycoprotein and is primarily present in the upper parts of articular cartilage. In all types of cartilages it is synthesized by chondrocytes. The glycoprotein is composed of five identical sulphide-linked subunits with an apparent Mr of about 100 kDa each. It is markedly anionic, probably due to a high content (about 30%) of aspartic and glutamic acid, and has also high contents of cysteine, giving an extensive disulphide bonding.

COMP and its characterstics is extensively described in (1). Further, the nucleotide and the deduced amino acid sequence of COMP is presented in (2). Furthermore, the model of COMP is shown in (8).

As hydroxyproline has not been found in COMP, COMP does not contain any collagenous region. From the carbohydrate composition, the presence of N-linked, but not of O-linked oligosaccharides, can be predicted. The presence of xylose, together with galactose amine and glucuronic acid in close to equimolar amounts, indicates substitution with chondroitin sulfate. The values of xylose, glucuronic acid and galactose amine suggests that each COMP subunit carries 3-4 chondroitin sulfate chains of 14-15 disaccharides each.

Thus, COMP is a known compound and has previously been described as being useful in diagnostic methods for estimating the degree of cartilage degradation in arthritic patients (9).

It is already known that autoimmune reactions to certain cartilage antigens present in the joint cartilage cab be used to induce rheumatoid arthritis in animal models. Collagen of different types (II, IX, and XI) and aggrecan have been classified as cartilage autoantigens inducing autoimmunity and rheumatoid arthritis in animal models. These antigens have also been used in efforts to treat rheumatoid arthritis.

However, it has not previously been shown that COMP is arthritogenic and therefore potentially of use in the treatment of rheumatoid arthritis. The inventors of the present invention have reached these results after experiments in which COMP has been added to rats. These experiments are described below.

The present invention therefore relates to the use of cartilage oligomeric matrix protein (COMP), or fragments or peptide analogues thereof, for the manufacture of a pharmaceutical composition for prevention or treatment of rheumatoid arthritis in a mammal, wherein the pharmaceutical composition is to be administered in an amount effective to prevent or treat the rheumatoid arthritis.

In one embodiment, the pharmaceutical composition comprises an RNA or DNA sequence capable of expressing the COMP or fragments or peptide analogues thereof, or a vector cell containing said RNA or DNA sequences.

The pharmaceutical compositions of the present invention optionally also comprise one or more substances selected from the group consisting of collagene II, collagene IX, collagene XI, and aggrecan, or fragments or analogues thereof.

The COMP of use in the present invention may be of human, bovine, or rat origin, produced by microorganisms or chemically synthesized.

### Figure relating to the Experiments

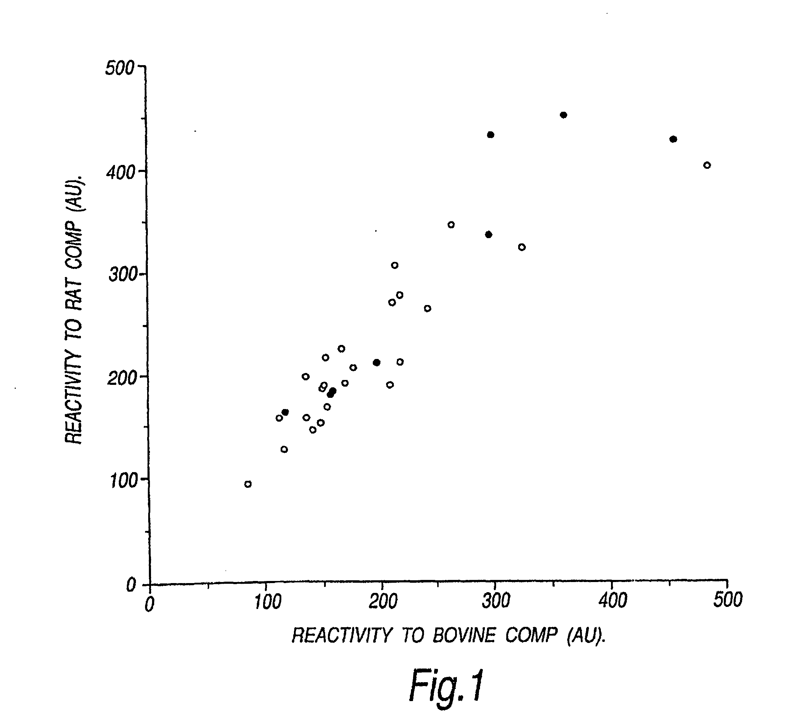

Fig. 1 shows the humoral reactivity to bovine COMP and rat COMP in sera from rats immunized with bovine COMP. An ELISA procedure was used to assess the humoral reactivity to COMA in sera from LEW.1AV1 (o) and F344 (•) rats, 16 and 38 days after immunization with 50-150 µg/rat of bovine COMP.

### Experiments

The rat strains LEW.1AV1(DA) and F344 were originally derived from Zentralinstitut für Versuchstierzucht, Hannover, Germany. Characteristics and genetics of these strains are described in Genetic monitoring of inbred strains of rats (3). All strains were bred at the Biomedical Center in Uppsala and housed there or at the Karolinska Hospital under specific pathogen-free conditions. They were free from pathogens as determined by a health monitoring program for rats (Nov. 1995) at the National Veterinary Institute in Uppsala, Sweden. The rats were kept in a 12 h light/dark cycle, housed in polystyrene cages containing wood shavings and had free access to water and rodent chow. Rats used in experiments were females aged 10-19 weeks. All procedures involving animals were performed according to the guidelines provided by the central board for animal experiments at the Swedish Department of Agriculture. The experiments were approved by the Ethical board for animal experiments in Stockholm-North.

### Induction of arthritis with bovine COMP and rat CII

Induction of arthritis with COMP was performed as follows: bovine COMP, prepared from cartilage (1), was dissolved in phosphate buffered saline (PBS) pH 7.4 at a concentration of 0.5 or 1.5 mg/ml. This solution was emulsified with an equal volume of incomplete Freund's adjuvant (Difco, Detroit) and 200 µl of the emulsion was injected intradermally at the base of the tail (50 or 150 µg/rat respectively). Induction of arthritis with collagen was performed as follows: rat collagen type II (CII), prepared from a chondrosarcoma (4, 5), was dissolved in 0,1 M acetic acid at a concentration of 1.5 mg/ml. This solution was emulsified with an equal volume of incomplete Freund's adjuvant and 200 µl of the emulsion was injected intradermally at the base of the tail (150 µg/rat).

### Evaluation of arthritis

Arthritis was assessed using a scale from 0-16, each of four paws being scored from 0-4 in which 0=no arthritis, 1-3=a summary of points as follows: swelling of the ankle-1 point, swelling of one or more intratarsal and/or metatarsal joints-1 point, and swelling of one or more interphalangeal joints-1 point, 4=swelling of all joints, i.e. the entire paw. The rats were examined every second to fourth day from day 10 until day38 after immunization.

### Preparation of draining lymph node cells

Inguinal lymph node cells from rats immunized with COMP and from non-immunized control rats were obtained by dissection of the lymph nodes and gentle grinding through a tea-strainer. The cell suspensions were washed three times in PBS pH 7.4.

### Measurement of cellular reactivity to bovine COMA: Proliferation

The inguinal lymph node cells were suspended at 1x10⁶ cells/ml in DMEM (X) supplemented with 5% FCS (Hyclone, Logan, UK), penicillin (100 u/ml), glutamine (2 nM) and streptomycin (100 µg/ml), all from Sigma, St Louis, MO. Cells from individual animals were plated in 96-well round-bottom cell culture plates (Nunc, Roskilde, Denmark), 0.2 ml per well. Antigens were added to triplicates of wells for each animal, dissolved in 10 µl PBS pH 7.4, to give the final concentrations: COMP, 10 µg/ml and 50 µg/ml, or ConA 5 µg/ml. As control, 10 µl of PBS were added to the wells. The cells were incubated for 96 hours in 37°C and 5% CO₂, and cell proliferation assessed by labelling proliferating cells in each well with 1 µCi of ³H-thymidine for the final 6 hours before cell harvest. Incorporation of label was determined by liquid scintillation counting.

### Purification of rat COMP

Rat COMP was prepared from Swarm rat chondrosarcoma (6). Briefly, frozen Swarm rat chondrosarcoma was homogenized in PBS containing 1 mM phenylmethanesulfonyl fluoride (PMSF) and 2 mM N-ethylmaleimide (NEM) followed by centrifugation for 30 min at 15 000 g. The homogenization and centrifugation was repeated twice, followed by extraction of COMP in the same buffer containing in addition 10 mM EDTA. The EDTA extract was diluted 1+1 in water and applied to an anion exchange column (20x1.6 cm) of DEAE Sepharose CL-6B (Pharmacia LKB Biotechnology) equilibrated with 10 mM tris containing 2 mM EDTA. The column was eluted with a gradient of 0-1.0 M NaCl in the same buffer. COMP containing fractions were concentrated by ultrafiltration (YM-10 filter, Amicon) and applied to a gel filtration column (50 x 1.6 cm) of Superose 6 (Pharmacia LKB Biotechnology), and eluted with PBS containing 2 mM EDTA.

### Quantitation of humoral anti-bovine COMP, anti-rat COMP and anti-rat CII immunity

IgG antibody titers to COMP or CII were determined using an enzyme-linked immunosorbent assay (ELISA). Maxisorp Micro-ELISA plates (Nunc, Roskilde, Denmark) were coated overnight at 4°C with 10 µg/ml of rat CII or 2-10 µg/ml of COMP in PBS pH 7.4. Individual sera were diluted, added and bound antibody was detected by incubation with alkaline phosphatase-conjugated goat anti-rat IgG, specific for heavy and light chains (Jackson Immuno-research Laboratories, West Groove, PA). The subsequent quantitation of bound enzyme was performed with a p-nitrophenyl-containing substrate buffer in an E-max spectrophotometer (Molecular Devices, Sunnyvale, CA). Data are presented as antibody units (AU) per ml, calculated by comparison with the linear portion of a standard curve using positive and pooled anti-CII serum or anti-COMP serum from LEW.1AV1 rats with given arbitrary values of 100 AU/ml respectively. Each plate contained positive reference serum, serum samples to be measured and negative serum controls in duplicates. Negative serum controls were individual sera from non-immunized LEW.1AV1 rats.

### RESULTS

### Arthritogenicity of bovine COMP in LEW.1AV1 and F344 rats

In the first experiment, six LEW.1AV1 rats were immunized with 50 µg COMP/rat (table 1). 15-19 days later, three of these (3/6) developed arthritis which mainly affected the hind ankles. The disease was self--limiting, there were no macroscopic signs of arthritis or ankylosis 38 days after immunization. In the second experiment, nine LEW.1AV1 rats and four age-matched F344 rats were immunized with 150 µg COMP/rat (Table 1). This higher dose of COMP resulted in a similar incidence of arthritis in the LEW.1AV1 rats (5/11) but the disease appeared earlier, 12-14 days after immunization. Furthermore, the arthritis was more servere and typically affected ankle joints and metatarsal joints in the hind paws, arthritis in finger joints and fore paws was also observed. Two rats were monitored clinically until 38 days after immunization at which time they had signs of ankylosis. In contrast to rats of the LEW.1AV1 strain, F344 rats appeared less susceptible to COMP-induced arthritis since only minimal signs of disease could be detected, at a late time-point 25-29 days after immunization, in one out of four rats.

### Humoral reactivity to bovine COMP in individual LEW.1AV1 and F344 rats, 16 days after immunization with bovine COMP

Analysis of individual sera demonstrated humoral reactivity to COMP in both F344 rats and LEW.1AV1 rats (Table 1). There was no correlation between presence of antibodies and arthritis.

### Humoral cross-reactivity between bovine COMP and rat COMP

Sera from LEW.1AV1 and F344 rats, immunized with bovine COMP, were tested for humoral reactivity to bovine COMP and rat COMP (Figure 1). The reactivity to bovine COMP correlated well to that against rat COMP in both rat strains, which demonstrates that the humoral immune response induced by immunization with bovine COMP is autoimmune.

### Humoral reactivity to COMP and CII in LEW.1AV1 rats with arthritis induced by immunization with COMP or CII

Sera from LEW.1AV1 rats immunized with incomplete Freund's adjuvant alone, or together with bovine COMP or rat CII respectively, were tested for humoral reactivity to both bovine COMP and rat CII. The results depicted in Table 2 demonstrate that reactivity to COMP and CII develops in LEW.1AV1 immunized with the respective antigens, and that there is no cross-reactivity between these two antigens. Interestingly, there were no signs of an anti-CII reactivity in rats with COMP-induced arthritis. No or minimal reactivity to COMP was detected in rats with CII-induced arthritis.

Two features of the humoral and cellular immune response in arthritic animals were of principal interest. Firstly, the humoral reactivity to COMP was apparently not accompanied by reactivity to rat collagen type II (CII). Thus, there is no evidence for immunological cross-reactivity between these two arthritogenic antigens, neither is there evidence for spreading of the humoral autoimmune response to rat CII. Secondly, a marked proliferative cellular immune response towards COMP was evident. This implies that the nature of T-cell mediated immune reactions towards COMP is more amenable to study than those to previously described cartilage autoantigens, such as rat CII which induces little or no in vitro T-cell proliferation (unpublished results).

In this study it was also demonstrated that while the LEW.1AV1 strain were permissive for COMA-induced arthritis, F344 rats appeared resistant. This indicates that susceptibility is genetically determined.

COMP is of interest as an arthritis-inducing antigen for several reasons. Firstly, COMP is present in the most superficial parts of the articular cartilage where it may be accessible to immune cells in the joints. Secondly, COMP is released into synovial fluid and serum of patients with early destructive arthritis (7), implying that this protein is also accessible to circulating immune cells. It has not earlier been reported whether an induced autoimmunity against COMP can cause arthritis such as in this present study, nor whether autoimmune reactions towards COMP occur in patients with arthritis.

Bovine and rat COMP used in these experiments were purified from cartilage such that no contamination of other proteins could be detected, as observed by the absence of other proteins than COMP on SDS-PAGE gels used for the biochemical characterization (data not shown). However, the existence of minor contaminants in the preparation may not be excluded, but such contaminants are unlikely to be responsible for arthritis induction since their concentrations would be orders of magnitude lower than that of other arthritogens used in rats. That contaminating collagen type II is not involved in arthritis development is also indicated by the lack of a humoral immune response to rat CII in rats immunized with the COMP preparation.

**Table. 1.**

| Development of arthritis and humoral reactivity to COMP in LEW.1AV1 rats and F344 rats after immunization with COMP. | | | | | | | |
|---|---|---|---|---|---|---|---|
| Rat Strain | # | Dose of OOMP (µg/rat) | Day of Onset | Maximal Severity 0-38 dpit | Severity 38 dpi | α-COMP (AU) 16 dpi | α-COMP (AU) 38 dpi. |
| LEW.1AV1 | a1 | 50 | 15 | 1 | 0 | nd** | 219 |
| (6) | a2 | 50 | - | 0 | 0 | nd | 153 |
| | a3 | 50 | - | 0 | 0 | nd | 213 |
| | a4 | 50 | - | 0 | 0 | nd | 114 |
| | a5 | SO | 19 | 2 | 0 | nd | 215 |
| | a6 | 50 | 19 | 3 | 0 | nd | 326 |
| | | | | | | | |
| LEW.1AV1 | b1* | 150 | 12-14 | 2 | na†† | 155 | na |
| (11) | b2* | 150 | 12-14 | 5 | na | 152 | na |
| | b3 | 150 | - | 0 | 0 | 210 | 219 |
| | b4* | 150 | 12-14 | 2 | na | 117 | na |
| | b5 | 150 | - | 0 | 0 | 148 | 168 |
| | b6 | 150 | 12-14 | 4 | 2 | 137 | 265 |
| | b7 | 150 | 12-14 | 7 | 6 | 142 | 483 |
| | b8 | 150 | - | 0 | 0 | 151 | 243 |
| | b9 | 150 | - | 0 | 0 | 85 | 178 |
| | b10* | 150 | - | 0 | na | 136 | na |
| | b11* | 150 | - | 0 | na | 170 | na |
| | | | | | | | |
| F344 | b12 | 150 | - | 0 | 0 | 157 | 298 |
| (4) | b13 | 150 | - | 0 | 0 | 199 | 300 |
| | b14 | 150 | - | 0 | 0 | 160 | 361 |
| | b15 | 150 | 26-29 | 1 | 0 | 118 | 453 |
| LEW.1AV1 and F344 rats were immunized with bovine COMP in incomplete Freund's adjuvant, the humoral reactivity was tested against bovine COMP by an ELISA procedure. #=animal number. †dpi=days post-immunization, *these rats were killed 16 dpi. **nd=not done. ^{††}na=not applicable because rats were killed 16 dpl. | | | | | | | |

**Table 2.**

| Humoral reactivity to COMA and collagen type II (CII) in LEW.1AV1 rats immunized with incomplete Freund's adjuvant (IFA) alone, or together with COMP or CII. | | | |
|---|---|---|---|
| Rats immunized with: | Maximal severity | α-Cll (AU) | α-COMP (AU) |
| IFA (6) | 0 | 0 | 0 |
| CII+IFA (5) | 7-12 | 61-223 | 0-2 |
| COMP+IFA (5) | 1-7 | 0 | 215-483 |
| Sera were obtained from rats which developed bovine COMP-induced arthritis (38 days post immunization, dpi), rat collagen-induced arthritis (28 dpi) or, as a control, from rats immunized with IFA (28 dpi). | | | |

**Table 3.**

| Proliferation of lymph node cells from LEW.1AV1 rats immunized with COMP, a comparison between unstimulated cells and cells stimulated with COMP or Con A. | | | | |
|---|---|---|---|---|
| Animal number | Arthritis Severity | Proliferation Control | (cpm) OOMP | stimulated by: Con A |
| 1 | 2 | 1506 | 12433 | 26315 |
| 2 | 5 | 2754 | 27014 | 37624 |
| 3 | 2 | 4541 | 25604 | 38135 |
| 4 | 0 | 3658 | 28820 | 40961 |
| Lymph node cells were prepared from four female LEW.1AV1 rats 16 days after immunization with 150µg/rat of bovine COMP emulsified in incomplete Freund's adjuvant. The cells were either unstimulated (control) or stimulated with bovine COMP (10 µg/ml) or with Con A (5 µg/ml). | | | | |

The expression "fragments" of COMP used throughout the application include any partial amino acid sequences or modified partial sequences thereof, i.e. those being shorter in length than COMP and/or containing any deletions/substitutions or modifications to the constituent amino acids, that are capable of inducing a biological response similar to COMP, i.e. the ability to suppress or eliminate T-cell or B-cell mediated and/or T-cell or B-cell dependent autoimmune response, or act in a manner so as to inhibit the arthritogenic effects of COMP. The expression "analogues" of COMP used throughout the application includes compounds (both peptides and peptide chemical compounds) that possess similar biological activity to COMP, i.e. the ability to eliminate or suppress the T-cell or B-cell mediated and/or T-cell or B-cell dependent autoimmune response, or act in a manner so as to inhibit the arthritogenic effects of COMP. Thus, the latter expression includes amino acid sequences which differ from the amino acid sequence of COMP by one or more amino acids, while retaining substantially equivalent biological activity, as well as chemical compounds which mimic the biological activity of COMP in its ability to suppress or alleviate the symptoms of rheumatoid arthritis.

The pharmaceutical composition comprising COMP or the fragments or peptide analogues thereof may be administrated to a mammal, preferably a human, in a number of known routes including oral, mucosal, nasal, rectal or vaginal route, or by inhalation or injection.

For oral administration the pharmaceutical composition may be e.g. tablets, pills, capsules, syrups, powders, granules, or solutions.

For oral administration the active compound may be admixed with an adjuvant or a carrier, e.g. lactose, saccharose, sorbitol, mannitol, starches such as potato starch, corn starch or amylopectin, cellulose derivatives, a binder such as gelatine or polyvinylpyrrolidone, and a lubricant such as magnesium stearate, calcium stearate, polyethylene glycol, waxes, paraffin, and the like, and then compressed into tablets. If coated tablets are required, the cores, prepared as described above, may be coated with a concentrated sugar solution which may contain e.g. gum arabic, gelatine, talcum, titanium dioxide, and the like. Alternatively, the tablet may be coated with a suitable polymer dissolved in a readily volatile organic solvent.

For the preparation of soft gelatine capsules, the compound may be admixed with e.g. a vegetable oil or polyethylene glycol. Hard gelatine capsules may contain granules of the compound using either the above mentioned excipients for tablets, e.g. lactose, saccharose, sorbitol, mannitol, starches, cellulose derivatives or gelatine. Also liquid or semisolid formulations of the drug may be filled into hard gelatine capsules.

Liquid preparations for oral application may be in the form of syrups or suspensions, for example solutions containing the compound, the balance being sugar and a mixture of ethanol, water, glycerol and propylene glycol. Optionally such liquid preparations may contain colouring agents, flavouring agents, saccharine and carboxymethylcellulose as a thickening agent or other excipients known to those skilled in art.

The pharmaceutical composition may also include pharmaceutically acceptable carriers, such as water, suspending agents, and emulsifying agents.

Also, mucosa binding compounds may be included in the pharmaceutical composition.

The exact dosages of the active ingredient, i.e. COMP or the biological active fragments or peptide analogues thereof, depend on the age, sex, and physical condition, i.e. the severity of rheumatoid arthritis, of the patient and on any concurrent treatments. However, the dosage has to be such that the symptoms of rheumatoid arthritis are prevented or alleviated.

In another embodiment the pharmaceutical composition according to the present invention comprises COMP or fragments or peptide analogues thereof in combination with one or more arthritogenic substances chosen from the group consisting of collagene II, IX, XI, and aggrecan, or fragments or analogues thereof.

COMP, and fragments or peptide analogues thereof, may be isolated from the naturally occuring material as described in (1) or may be expressed from the nucleotide sequence given for COMP in (2). The skilled man will be aware as to how such expression may be obtained and how such fragments or peptide analogues may be prepared from the nucleotide sequence. COMP and its fragments and peptide analogues may also be chemically synthesized using the deduced amino acid sequence given in (2).

The present invention therefore also relates to the use of COMA as described above, wherein the pharmaceutical composition comprises RNA or DNA sequences expressing COMP or fragments or peptide analogues thereof, or a vector cell containing said RNA or DNA sequences. Thus, the RNA or DNA sequences are administrated to the mammal either directly or via the vector cell.

COMP or the fragments or peptide analogues thereof may have any origin, but has preferably human, rat, or bovine origin. It may be prepared by purification from any of these origins by means of standard methods. It can also be expressed by a microorganism or be chemically synthesized, as described above. According to the present invention COMP or fragments or peptide analogues thereof may be added in a pharmaceutical composition to be administrated to mammals in order to prevent or alleviate the symptoms of rheumatoid arthritis. Thus, when the pharmaceutical composition according to the present invention is to be administrated to a mammal, preferably a human, having rheumatoid arthritis, COMP or the fragments or peptide analogues thereof acts as a kind of tolerogen and thereby induces immunological tolerance.

Further, COMP or fragments or peptide analogues thereof may also be used to prevent arthritic conditions, preferably rheumatoid arthritis by administration to a mammal suspected to develop such arthritic condition, e.g. due to genetical predictions. In such a case, the disturbed balance due to autoimmunological reactions will never appear, nor the arthritic condition.

### REFERENCES

1 Hedbom E, Antonsson P, Hjerpe A, Aeschlimann D, Paulsson M, Edson R-P, Sommarin Y, Wendel M, Oldberg Å, Heinegård D: Cartlage matrix proteins. An acidic oligomeric protein (COMP) detected only in cartilage. J Biol Chem 267:6132-6136, 1992
2 Oldberg Å, Antonsson P, Lindblom K, Heinegård D: COMP (cartilage oligomeric matrix protein) is structurally related to the thrombospondins. J Biol Chem 267:22346-22350, 1992
3 Hedrich HJ. (ed) Genetic monitoring of inbred strains of rats. Gustav Fischer Verlag, New York, 1990
4 Smith BD, Martin GR, Miller EJ, Dorfman A, Swarm R: Nature of the collagen synthesized by a transplanted chondocarcoma. Arch Biochem Biophys 166:181-186, 1975
5 Andersson ME, Holmdahl R: Analysis of type II collagen-reactive T-cells in the mouse. 1 Different regulation of autoreactive versus non-autoreactive anti-type II collagen T-cells in the DBA/1 mouse. Eur J Immunol 20:1061-1066, 1990
6 Mörgelin M, Heinegård D, Engel J, Paulsson: Electron microscopy of native cartilage oligomeric matrix protein purified from the Swarm rat chondrocarcoma reveals a five-armed structure. J Biol Chem 267:6137-6141, 1992
7 Månsson B, Carey D, Alini M, Ionescu M, Rosenberg LC, Poole AR, Heinegård D, Saxne T: Cartilage and bone metabolism in rheumatoid arthritis. Differences between rapid and slow progression of disease identified by serum markers of cartilage metabolism. Br J Rheum 34:306-310, 1995
8 Mörgelin M, Heinegård D, Engel J, Paulsson M: Electron microscopy of native cartilage oligomeric matrix protein purified from the swarm rat chondrosarcoma reveals a five-armed structure, the Journal of Biological Chemistry, Vol. 267, No. 9, March 25, Fig. 7.
9 Månsson B, Carey D, Alini M, Ionescu M, Rosenberg LC, Poole AR, Heinegard D, Saxne T: Cartilage and bone metabolism in rheumatoid arthritis differences between rapid and slow progression of disease identified by serum markers of cartilage metabolism. J Clin Invest, Vol. 95, 1995, p 1071-1077

## Claims

1. Use of cartilage oligomeric matrix protein (COMP), or fragments or peptide analogues thereof, for the manufacture of a pharmaceutical composition for prevention or treatment of rheumatoid arthritis in a mammal, wherein the pharmaceutical composition is to be administrated in an amount effective to prevent or treat rheumatoid arthritis

2. Use according to claim 1, wherein the pharmaceutical composition comprises RNA or DNA sequences expressing the COMP or fragments or analogues thereof, or a vector cell containing said RNA or DNA sequences.

3. Use according to claim 1 or 2, wherein in the pharmaceutical composition also comprises one or more substances selected from the group consisting of collagene II, collagene IX, collagene XI, and aggrecan, or fragments or analogues thereof.

4. Use according to claim 1, wherein the COMP has human, bovine, or rat origin, or has been produced by microorganisms or has been chemically synthesized.

5. Use according to any one of the preceding claims, wherein the pharmaceutical composition is administrated by the oral, mucosal, nasal, rectal or vaginal route, or by inhalation or injection.

6. Use according to claim 1, wherein the mammal is a human.

## Patentansprüche

1. Verwendung des Proteins COMP (Cartilage Oligomeric Matrix Protein) oder von Fragmenten oder Peptidanalogen davon zur Herstellung einer pharmazeutischen Zusammensetzung zur Vorbeugung bzw. Behandlung von rheumatoider Arthritis in einem Säuger, wobei die pharmazeutische Zusammensetzung in einer zur Vorbeugung bzw. Behandlung von rheumatoider Arthritis wirksamen Menge zu verabreichen ist.

2. Verwendung nach Anspruch 1, wobei die pharmazeutische Zusammensetzung RNA- oder DNA-Sequenzen, die das COMP oder Fragmente oder Analoge davon exprimieren, oder eine diese RNA- oder DNA-Sequenzen enthaltende Vektorzelle umfaßt.

3. Verwendung nach Anspruch 1 oder 2, wobei die pharmazeutische Zusammensetzung ebenfalls eine oder mehrere Substanzen, ausgewählt aus der Gruppe bestehend aus Kollagen II, Kollagen IX, Kollagen XI und Aggrecan, oder Fragmente oder Analoge davon umfaßt.

4. Verwendung nach Anspruch 1, wobei das COMP aus dem Menschen, dem Rind oder der Ratte stammt oder von Mikroorganismen produziert worden oder chemisch synthetisiert worden ist.

5. Verwendung nach einem der vorhergehenden Ansprüche, wobei die pharmazeutische Zusammensetzung auf oralem, mucosalem, nasalem, rektalem oder vaginalem Wege oder durch Inhalation oder Injektion verabreicht wird.

6. Verwendung nach Anspruch 1, wobei es sich bei dem Säuger um einen Menschen handelt.

## Revendications

1. Utilisation d'une protéine de matrice oligomère de cartilage (COMP), ou de fragments ou analogues peptidiques de celle-ci, pour la fabrication d'une composition pharmaceutique destinée à la prévention ou au traitement de la polyarthrite rhumatoïde chez un mammifère, dans laquelle la composition pharmaceutique est à administrer en quantité efficace pour prévenir ou traiter la polyarthrite rhumatoïde.

2. Utilisation selon la revendication 1, dans laquelle la composition pharmaceutique comprend des séquences d'ARN ou d'ADN exprimant la COMP ou des fragments ou analogues peptidiques de celle-ci, ou une cellule vectorielle contenant lesdites séquences d'ARN ou d'ADN.

3. Utilisation selon la revendication 1 ou 2, dans laquelle la composition pharmaceutique comprend aussi une ou plusieurs substances choisies dans le groupe constitué par le collagène II, le collagène IX, le collagène XI et l'aggrécan, ou leurs fragments ou analogues.

4. Utilisation selon la revendication 1, dans laquelle 1a COMP a une origine humaine, bovine ou murine, ou a été produite par des microorganismes ou a été synthétisée par voie chimique.

5. Utilisation selon l'une quelconque des revendications précédentes, dans laquelle la composition pharmaceutique est administrée par voie orale, par les muqueuses, par voie nasale, par voie rectale ou par voie vaginale, ou par inhalation ou injection.

6. Utilisation selon la revendication 1, dans laquelle le mammifère est un être humain.
